**Europäisches Patentamt**

**European Patent Office**

**Office européen des brevets**

(19)

(11) Veröffentlichungsnummer: **0 034 826**
**A1**

(12) # EUROPÄISCHE PATENTANMELDUNG

(21) Anmeldenummer: **81101267.3**

(22) Anmeldetag: **21.02.81**

(51) Int. Cl.³: **A 61 N 1/40**
**A 61 N 5/04**
**//F16M11/40**

(30) Priorität: **23.02.80 DE 8004905 U**

(43) Veröffentlichungstag der Anmeldung:
**02.09.81 Patentblatt 81/35**

(84) Benannte Vertragsstaaten:
**BE DE GB NL**

(71) Anmelder: **Erbe Elektromedizin GmbH. & Co.,KG**
**Postfach 1420**
**D-7400 Tübingen(DE)**

(72) Erfinder: **Farin, Günter**
**Kapellenweg 15**
**D-7400 Tübingen-Hirschau(DE)**

(72) Erfinder: **Fischer, Klaus**
**Tulpenweg 9**
**D-7306 Denkendorf(DE)**

(74) Vertreter: **Endlich, Fritz, Dipl.-Phys.**
**Postfach**
**D-8034 Germering(DE)**

(54) **Elektromedizinisches Wärmetherapiegerät.**

(57) Es wird ein Haltearm für ein elektromedizinisches Wärmetherapiegerät beschrieben, der mindestens entlang eines Teils seiner Länge eine biegsame Wendelstruktur aufweist, welche ein Fixierung des Haltearms mit einem daran angeordneten Applikator in jeder eingestellten Verbiegungslage durch mechanische Haftreibung ermöglicht. Die elektrische Zuführleitung ist in den Haltearm integriert. Bei Haltearmen für Kurzwellen-Wärmtherapiegeräte weisen die Haltearme eine Umhüllung aus flexiblem Isoliermaterial auf und die Wendelstruktur dient als Zuführleitung für den hochfrequenten elektrischen Strom. Applikatoren können in besonders einfacher Weise ausgewechselt werden, da bei der mechanischen Befestigung gleichzeitig die elektrische Verbindung hergestellt wird.

FIG.1

FIG.2

## Elektromedizinisches Wärmetherapiegerät

Die Erfindung betrifft ein elektromedizinisches Wärmetherapiegerät mit einer elektrisch isolierten Zuführleitung für einen an einem beweglichen Haltearm angeordneten Applikator.

Es sind bereits seit längerer Zeit Dezimeterwellen- und Mikrowellen-Therapiegeräte bekannt, die einen an dem Gerät angelenkten Haltearm aufweisen, damit der Applikator in eine geeignete Lage relativ zu dem zu bestrahlenden Patienten bewegt werden kann. Je nach der durchzuführenden Behandlung kann der an dem Haltearm angeordnete Applikator durch einen anderen Applikator ersetzt werden, beispielsweise durch einen Rundfeldstrahler, einen Langfeldstrahler oder einen Fokusstrahler.

Um den Applikator in eine geeignete Lage relativ zu dem Patienten bringen zu können, sind bereits zahlreiche bewegliche Konstruktionen von Haltearmen bekannt, die beispielsweise aus zwei Auslegern, einem Ansatzstück am Gerät und einem Anschlußteil für den Applikator bestehen können. Ein derartiger Haltearm kann in insgesamt sechs Gelenken beweglich sein, so daß der Applikator bei ausreichender Geschicklichkeit der Bedienungsperson praktisch in jede gewünschte Position gebracht werden kann.

Bei diesen bekannten Konstruktionen wird als nachteilig angesehen, daß sie entweder konstruktiv verhältnismäßig aufwendig sind und zum Einjustieren ein beträchtlicher Arbeitsaufwand erforderlich ist, oder daß bei einfacherer Konstruktion des Haltearms der Applikator nicht in jede gewünschte Position relativ zu dem Patienten bewegt werden kann. Außerdem ist das erforderliche Kabel für die Energiezufuhr in vielen Fällen störend, zumal es bei teleskopartig verkürzbaren Haltearmen mit einer verhältnismäßig großen Schlaufe herabhängt. Deshalb kann es auch in gewissen Fällen vorkommen, daß das Kabel geknickt oder eingeklemmt wird, so daß auch Beschädigungen des Kabels verursacht werden können.

Bei Wärmetherapiegeräten für Kurzwellen bestehen außer den bereits genannten Schwierigkeiten noch zusätzliche Schwierigkeiten, weil an derartigen Geräten im allgemeinen zwei Haltearme vorgesehen sind, an deren Enden jeweils eine Kondensatorfeld-Elektrode angeordnet ist, so daß auch zwei Kabel für die Zuführleitung erforderlich sind. Die einadrigen oder zweiadrigen Kabel zur Zuleitung des hochfrequenten elektrischen Strom vom Hochfrequenzgenerator zu den Applikatoren können elektrisch nicht abgeschirmt werden, so daß diese Kabel ähnlich wie die Kondensatorfeld-Applikatoren hochfrequente magnetische Felder abstrahlen. Werden diese Kabel zu nahe am Körper des Patienten vorbeigeführt, oder berühren diese Kabel den Patienten, so können zwischen Kabel und Patient derart hohe elektrische Feldstärken entstehen, daß daraus am Patienten Verbrennungen resultieren.

Da bei allen bisher bekannten Kurzwellen-Therapiegeräten die beiden Kabel zwischem dem Hochfrequenzgenerator und den Applikatoren freiliegend sind, kommt es auch verhältnismäßig oft vor, daß die beiden Kabel sich während der Bestrahlung zufällig berühren. An den Berührungsstellen der beiden Kabel treten dann hohe elektrische Feldstärken auf, die zu einer Verbrennung des Isoliermaterials der Kabelumhüllung führen können. Um die dadurch bedingten Gefahren möglichst weitgehend zu vermeiden, war es bei den bisher bekannten besten Konstruktionen derartiger Geräte üblich, Halteklammern an den Haltearmen anzuordnen, mit denen die Kabel in der Nähe der Haltearme gehaltert und geführt werden. Bei extremen Verstellungen der Haltearme verbleiben aber immer noch erhebliche Gefährdungen der genannten Art. Dies kann auch psychologisch nachteilig für den Patienten sein, weil die zusätzlichen Kabel eine Unsicherheit verursachen.

Es ist deshalb Aufgabe der Erfindung, bei elektromedizinischen Wärmetherapiegeräten bestehende Schwierigkeiten und auch gegebenenfalls bestehende Gefährdungen der beschriebenen Art durch Schaffung einer verbesserten Konstruktion eines beweglichen Haltearms möglichst weitgehend zu verringern. Diese Aufgabe wird erfindungsgemäß durch den Gegenstand des Patentanspruchs 1 gelöst. Vorteilhafte Weiterbildungen der Erfindung sind Gegenstand der Unteransprüche.

Durch die Erfindung wurde deshalb ein elektromedizinisches Wärmetherapiegerät geschaffen, bei dem der oder die Haltearme, die eine biegsame Wendelstruktur an sich bekannter Konstruktion aufweisen können, und die deshalb ohne weiteres in jede beliebige Position des Applikators verbogen werden können, ohne daß dafür eine ausreichende Geschicklichkeit oder ein besonderer Zeitaufwand erforderlich ist, wie es beispielsweise bei ausreichend beweglichen Haltearmen mit mehreren Gelenken der Fall ist, die einzeln eingestellt werden müssen. Durch die in den Haltearm integrierte Zuführleitung ist auch das Auswechseln der Applikatoren im Vergleich zu bekannten Wärmetherapiegeräten vereinfacht, weil den bekannten Konstruktionen zwei Arbeitsgänge erforderlich sind, weil erstens die Kabel von den Applikatoren gelöst werden müssen und zweitens die Applikatoren vom Haltearm gelöst werden müssen, während bei einem Wärmetherapiegerät gemäß der Erfindung lediglich eine einzige Rastkupplung oder dergleichen Einrichtung betätigt werden muß, um sowohl die mechanische als auch die elektrische Verbindung herzustellen bzw. zu unterbrechen. Außerdem wird der störende Einfluß durch mehr oder weniger unkontrolliert herabhängende Zuführungskabel vermieden, was insbesondere bei Anwendung der Erfindung auf Kurzwellen-Wärmetherapie die Möglichkeit des Auftretens einer Verbrennungsgefahr erheblich herabsetzt.

Anhand der Zeichnung soll die Erfindung beispielsweise näher erläutert werden. Es zeigen:

Fig. 1    einen Abschnitt eines teilweise im Schnitt dargestellten Haltearms für ein Wärmetherapiegerät gemäß der Erfindung;

Fig. 2    eine perspektivische Ansicht eines Kurzwellen-Wärmetherapiegeräts gemäß der Erfindung;

Fig. 3    eine Steckverbindung für eine Spulenfeldelektrode bei einem Kurzwellen-Wärmetherapiegerät gemäß der Erfindung;

Fig. 4    eine Schnittansicht eines Ausführungsbeispiels eines Haltearms für ein Kurzwellen-Wärmetherapiegerät gemäß der Erfindung; und

Fig. 5    eine Schnittansicht eines Ausführungsbeispiels eines Haltearms für ein Mikrowellen-Wärmetherapiegerät gemäß der Erfindung.

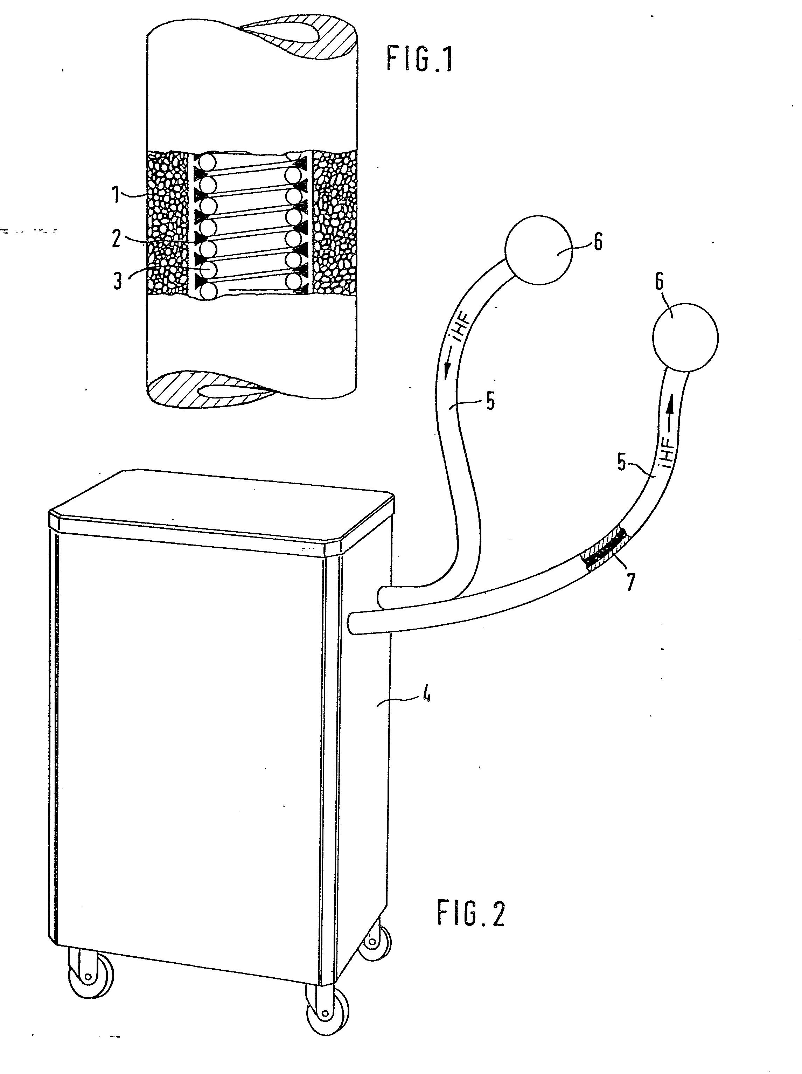

Das in Fig. 1 dargestellte Ausführungsbeispiel der Erfindung betrifft einen Haltearm für ein Kurzwellen-Wärmetherapiegerät. Der Haltearm weist eine Umhüllung 1 aus flexiblem Isoliermaterial auf, die zweckmäßigerweise durch einen dicken Schaumstoffschlauch gebildet ist, der eine glatte geschlossene Oberfläche aufweist. Diese aus flexiblem Isoliermaterial bestehende Umhüllung umgibt eine aus einer Metallwendel 3 und einer metallischen Haltewendel 2 bestehende Anordnung. Die Metallwendel 3, die aus Draht mit einem kreisförmigen Querschnitt hergestellt ist, ist durch die Haltewendel 2, die aus Draht mit einem dreieckförmigen Querschnitt hergestellt ist, derart eng umwickelt, daß der Haltearm durch mechanische Haftreibung zwischen der Haltewendel 2 und der Metallwendel 3 nach Einstellung in eine gewünschte Verbindungslage fixiert ist.

Fig. 2 zeigt ein Ausführungsbeispiel eines elektromedizinischen Kurzwellen-Wärmetherapiegeräts mit zwei Haltearmen 5, welche die in Verbindung mit Fig. 1 erläuterte Ausbildung besitzen. An den beiden freien Enden der an dem Gerät 4 angeordneten Haltearme 5 ist je eine Kondensatorfeld-Elektrode 6 befestigt. Die Haltearme 5 besitzen eine für alle praktisch interessierenden Anwendungsfälle ausreichende Länge. Wenn man einen Haltearm 5 mit beiden Enden in einem ausreichenden Abstand voneinander anfaßt, kann man die daran angeordnete Elektrode 6 mit einem einzigen Griff in die für die Behandlung erforderliche Relativlage zu dem Patienten bringen.

Bei einem Kurzwellen-Wärmetherapiegerät dienen die Metallwendel 3 und die metallische Haltewendel 2 als Zuführleitung 7 für den hochfrequenten elektrischen Strom.

Wenn der in Fig. 1 dargestellte Haltearm für ein Dezimeterwellen- oder Mikrowellen-Wärmetherapiegerät verwendet wird, kann die Zuführleitung in dem Innenraum der Metallwendel 3 angeordnet werden. Als Zuführleitung findet dann vorzugsweise ein Koaxialkabel Verwendung.

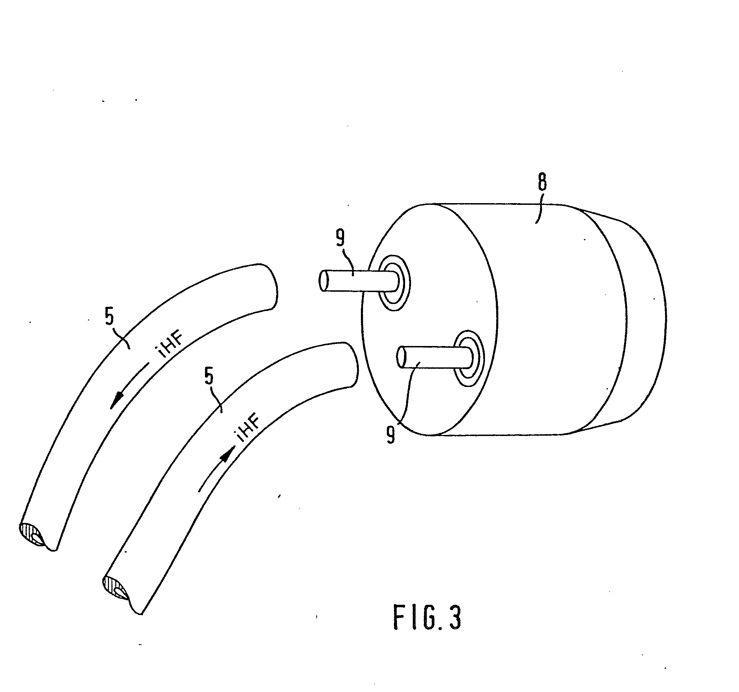

Fig. 3 zeigt eine Steckverbindung für eine Spulenfeld-Elektrode bei einem Kurzwellen-Wärmetherapiegerät gemäß der Erfindung. Die Spulenfeld-Elektrode 8 ist mit zwei elektrischen Kontakten 9 versehen, die in die beiden Haltearme 5 eingesteckt werden können.

Der hochfrequente elektrische Strom fließt durch den einen Haltearm 5 in die Spulenfeld-Elektrode hinein und über den anderen Haltearm 5 aus der Spulenfeld-Elektrode 8 heraus, zurück zu dem
Hochfrequenzgenerator in dem Gerät 4 in Fig. 2. Die Konstruktionen
derartiger, im Handel erhältlicher Geräte der Anmelderin und anderer Hersteller sind bekannt. Da Spulenfeld-Elektroden schwerer
sind als Kondensatorfeld-Elektroden ist der Anschluß der Spulenfeld-Elektroden an zwei Haltearmen mechanisch stabiler.

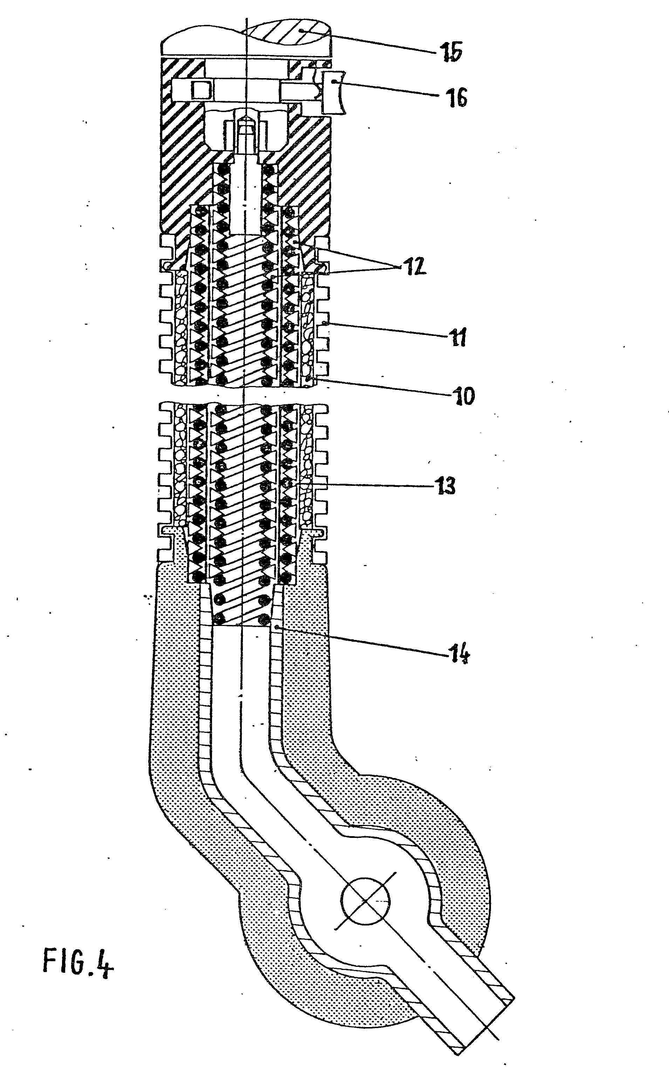

Fig. 4 zeigt ein weiteres Ausführungsbeispiel eines Haltearms
für ein Kurzwellen-Wärmetherapiegerät, bei dem eine doppelte Anordnung von ineinandergeschobenen Haltewendeln 12 und Metallwendeln 13 vorgesehen ist, die von einem geschäumten Kunststoffschlauch 10 und einer äußeren Umhüllung 11 aus Kunststoff umgeben
sind. Mit den metallischen Wendeln steht eine elektrische Zuführleitung in Verbindung, die an den Hochfrequenzgenerator in dem
Gerät angeschlossen ist.

Eine doppelte Wendelstruktur ergibt den Vorteil, daß sie eine
größere Tragkraft hat. Bei dem dargestellten Ausführungsbeispiel
ist zwischen der äußeren und der inneren Wendelstruktur ein
Kunststoffschlauch angeordnet, durch den eine Geräuschdämpfung
beim Verbiegen bewirkt wird, wenn die metallischen Windungen
direkt aneinander angreifen würden.

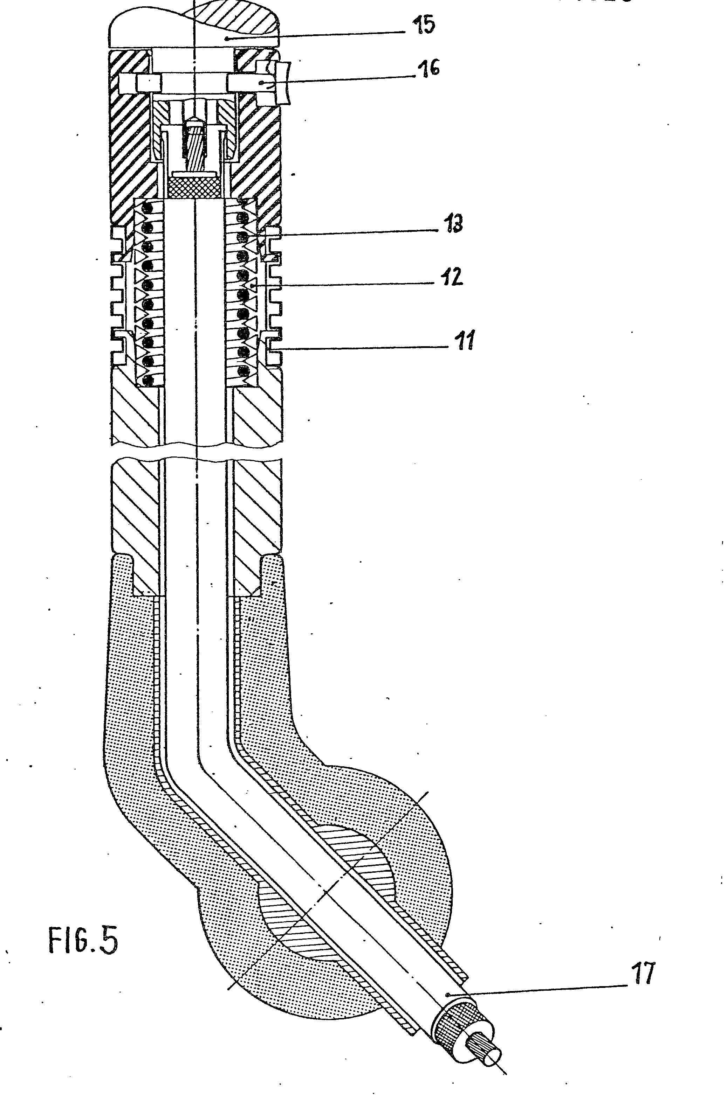

Als Applikator 15 ist bei dem Ausführungsbeispiel in Fig. 5 ein Fokusstrahler vorgesehen, dessen vorragendes Ende zylindrisch ausgebildet ist und dessen Außendurchmesser etwa gleich demjenigen des
Haltearms ist. Der Applikator 15 ist durch eine am freien Ende
des Haltearms vorgesehene Rastkupplung 16 sowohl mechanisch als
auch elektrisch verbindbar.

Das in Fig. 5 dargestellte Ausführungsbeispiel zeigt einen Haltearm für ein Dezimeterwellen- oder Mikrowellen-Wärmetherapiegerät,
an dem ebenfalls eine Rastkupplung 16 vorgesehen ist, mit dem der
Applikator 15 in einem einzigen Arbeitsgang elektrisch und mechanisch anschließbar beziehungsweise entfernbar ist. Bei diesem
Ausführungsbeispiel ist nur eine Wendelstruktur mit einer Metallwendel 13 und einer Haltewendel 12 vorgesehen. Ein geschäumter
Kunststoffschlauch, der bei dem Ausführungsbeispiel in Fig. 4 zu
Isolierzwecken dient, ist in diesem Fall nicht erforderlich, und es ist lediglich eine äußere Umhüllung 11 aus Kunststoff vorgesehen. Als Zuführleitung dient ein Koaxialkabel 17.

Bei den beiden in Fig. 4 und 5 dargestellten Ausführungsbeispielen ist der Haltearm an seinem am Gerät angelenkten Ende
durch ein starres Rohrstück gebildet. Durch die starre Ausbildung
des angelenkten Endes des Haltearms wird verhindert, daß beim
Bewegen oder Verbiegen des Haltearms die Wendelstruktur am
geräteseitigen Ende zu stark verformt beziehungsweise gedrückt
wird, weil hier das Drehmoment am größten ist. Bei geeigneter
Länge des starren Rohrstücks wird das Drehmoment besser in das
Drehgelenk am geräteseitigen Ende des Haltearms eingeleitet, so
daß sich erst das Drehgelenk bewegt, bevor die Haltewendel sich
verbiegt.

Die beiden Ausführungsbeispiele in Fig. 4 und 5 zeigen die Kabelführung beziehungsweise die Stromführung in dem geräteseitigen
Gelenk. Bei dem Kurzwellen-Wärmetherapiegerät in Fig. 4 ist es
wichtig, daß die HF-Stromführung gut gegen die metallischen Gelenkteile isoliert ist und einen großen Abstand von diesen hat,
damit die elektrische Kapazität zwischen Stromleiter und Metallteilen möglichst klein ist. Wenn die Kapazität der Stromleiter
zu den Metallteilen des Gerätegehäuses zu groß wäre, würde hierüber ein zu großer kapazitiver HF-Strom fließen.

Die äußere Umhüllung 11 besteht aus einem gerippten Kunststoffschlauch, der mit den starren Enden des Haltearms in der aus den
beiden Figuren ersichtlichen Weise verankert ist. Die Verankerung
des Kunststoffschlauchs mit den starren Enden des Haltearms erfolgt zweckmäßigerweise mit den dargestellten federnden Klammern,
die von innen in das Profil der Umhüllung hineingreifen und diese
festhalten.

Ein Unterschied zwischen dem Ausführungsbeispiel in Fig. 4 und 5
besteht auch darin, daß bei dem Haltearm für das Mikrowellen-
Wärmetherapiegerät in Fig. 5 die Wendelstruktur 12,13 nur entlang einer verhältnismäßig kurzen Länge vorgesehen ist, was für
viele Anwendungsfälle völlig ausreicht, weil durch das Drehgelenk in Verbindung mit einer Wendelstruktur von beispielsweise
10 cm Länge eine ausreichende Beweglichkeit gewährleistet ist.

ANSPRÜCHE

1. Elektromedizinisches Wärmetherapiegerät mit einer elektrisch isolierten Zuführleitung für einen an einem beweglichen Haltearm angeordneten Applikator, d a d u r c h  g e k e n n - z e i c h n e t, daß der Haltearm mindestens entlang eines Teils seiner Länge eine biegsame Wendelstruktur aufweist, welche eine Fixierung des Haltearms mit einem daran angeordneten Applikator in jeder eingestellten Verbiegungslage durch mechanische Haftreibung ermöglicht, und daß die elektrische Zuführleitung in dem Haltearm integriert ist.

2. Elektromedizinisches Wärmetherapiegerät nach Anspruch 1, d a d u r c h  g e k e n n z e i c h n e t, daß der Haltearm zumindest an seinem einen, an dem Gerät angelenkten Ende durch ein starres Rohrstück gebildet ist.

3. Elektromedizinisches Wärmetherapiegerät nach Anspruch 1 oder 2, d a d u r c h  g e k e n n z e i c h n e t, daß der Applikator durch eine am freien Ende des Haltearms vorgesehene Rastkupplung sowohl mechanisch als auch elektrisch verbindbar ist.

4. Elektromedizinisches Wärmetherapiegerät nach einem der vorhergehenden Ansprüche, d a d u r c h  g e k e n n z e i c h - n e t, daß die Zuführleitung in dem Innenraum der Wendelstruktur angeordnet ist.

5. Elektromedizinisches Wärmetherapiegerät nach Anspruch 4, d a d u r c h  g e k e n n z e i c h n e t, daß die Zuführleitung ein Koaxialkabel ist.

6. Elektromedizinisches Wärmetherapiegerät nach einem der vorhergehenden Ansprüche, d a d u r c h  g e k e n n z e i c h - n e t, daß eine Zuführleitung für Mikrowellen vorgesehen ist.

7. Elektromedizinisches Wärmetherapiegerät mit zwei Haltearmen nach einem der vorhergehenden Ansprüche 1 bis 3 und einer Zuführleitung für Kurzwellen, d a d u r c h  g e k e n n - z e i c h n e t, daß die biegsame Wendelstruktur durch eine von einer Metallwendel (3) eng umwickelte metallische Haltewendel (2) gebildet ist, daß die Haltearme (5) eine Umhüllung
(1) aus flexiblem Isoliermaterial aufweisen, und daß die beiden Wendeln (2,3) als Zuführleitung für den hochfrequenten
elektrischen Strom vorgesehen sind.

8. Elektromedizinisches Wärmetherapiegerät nach Anspruch 7,
d a d u r c h  g e k e n n z e i c h n e t ,  daß die Wendelstruktur aus zwei ineinandergeschobenen Sätzen von Haltewendeln (12) und Metallwendeln (13) besteht.

FIG.1

FIG.2

0034826

FIG.3

0034826

FIG.4

0034826

FIG.5

**0034826**

Nummer der Anmeldung

EP 81 10 1267

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 1 308 053 (COMPAGNIE FRAN-CAISE DE MICRO-ONDES)<br>* Seite 2, linke Spalte, Abschnitte 4,5; Seite 4, linke Spalte, letzter Abschnitt und rechte Spalte, Zeilen 1-7 * | 1,2,4-6 |
| | -- | |
| | US - A - 1 746 379 (CAMPBELL)<br>* Seite 2, Zeilen 24-28 * | 3 |
| | -- | |
| | FR - A - 1 073 173 (BILLIOQUE)<br>* Linke Spalte, letzter Abschnitt; Figur 1 * | 7 |
| | -- | |
| | DE - C - 282 354 (BLUMER)<br>* Zeilen 1-4; Figur * | 7 |
| | -- | |
| A | DE - C - 859 045 (KOCH)<br>* Seite 2, Zeilen 60-65, 73-85 * | 1 |
| | ---- | |

**KLASSIFIKATION DER ANMELDUNG (Int. Cl.)**

A 61 N 1/40
5/04//
F 16 M 11/40

**RECHERCHIERTE SACHGEBIETE (Int. Cl.³)**

A 61 N 1/40
1/06
5/04
F 21 V 21/32
F 16 M 11/40
A 61 N 5/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 25-05-1981 | SIMON |

EPA form 1503.1 06.78